## Europäisches Patentamt
## European Patent Office
## Office européen des brevets

(19)

(11) Publication number: **0 036 022**
**B1**

(12)

# EUROPEAN PATENT SPECIFICATION

(45) Date of publication of patent specification: **04.04.84**

(51) Int. Cl.³: **A 61 N 1/42**

(21) Application number: **80901075.4**

(22) Date of filing: **28.05.80**

(86) International application number:
**PCT/JP80/00115**

(87) International publication number:
**WO 81/00357 19.02.81 Gazette 81/5**

(54) **MAGNETIC MEMBER FOR MEDICAL CURE.**

(30) Priority: **06.08.79 JP 100519/79**
**24.01.80 JP 7860/80**

(43) Date of publication of application:
**23.09.81 Bulletin 81/38**

(45) Publication of the grant of the patent:
**04.04.84 Bulletin 84/14**

(84) Designated Contracting States:
**DE FR GB NL**

(73) Proprietor: **URAGAMI, Hideaki**
**12-12, Mefugaoka**
**Takarazuka-shi Hyogo 665 (JP)**

(72) Inventor: **URAGAMI, Hideaki**
**12-12, Mefugaoka**
**Takarazuka-shi Hyogo 665 (JP)**

(74) Representative: **Morgan, James Garnet et al,**
**MANITZ FINSTERWALD & ROTERMUND Robert-Koch-Strasse 1**
**D-8000 München 22 (DE)**

(56) References cited:
**DE - A - 2 235 015**
**DE - A - 2 321 485**
**FR - A - 373 320**
**FR - A - 2 285 112**
**FR - A - 2 467 588**
**JP - A - 49 001 092**
**JP - U - 52 046 595**
**US - A - 4 022 189**

**PATENT ABSTRACTS OF JAPAN, vol. 3, no. 108**
**(M-72) 11-09-1979 page 101M72**

Courier Press, Leamington Spa, England.

## Magnetic member for medical cure

The present invention relates to a magnetic medical treatment device comprising a flat magnetic element mounted upon the sticky surface of a sticking pad, the flat element being provided with a plurality of projections facing away from the sticky surface.

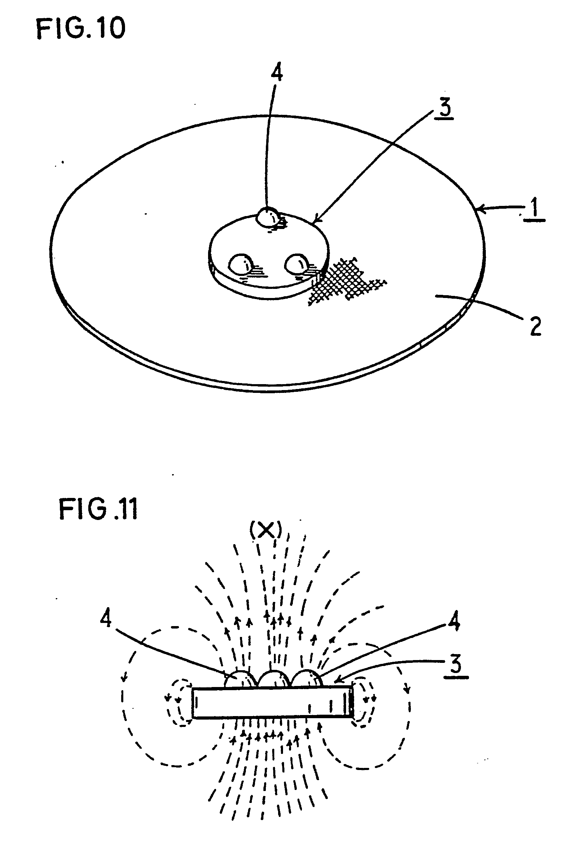

Devices of this kind are known and the conventional arrangement is illustrated in Figs. 10 and 11 of the accompanying drawings. In use the magnetic element (3) is stuck to the patient's skin by means of the sticky surface of the circular sticking pad surrounding the magnetic element. The projections which are provided near the centre of the magnetic element stimulate acupoints and meridians, thereby providing a "finger pressure effect" and lines of magnetic force simultaneously penetrate through the skin thereby providing a magnetic medical treatment effect. The projections provided upon the surface of said magnetic element concentrate the lines of magnetic force at the positions of each said projection.

A disadvantage of the known magnetic medical treatment device is that an insufficient medical treatment effect is achieved because the action of the lines of magnetic force is weak.

In order to make the reasons for this clear, the inventor of the present invention observed the state of lines of magnetic force in the conventional magnetic element to find the following phenomena:

a. lines of magnetic force tend to be concentrated onto projections on a flat magnetic press element.

b. Lines of magnetic force tend to be concentrated along a periphery of the flat portion of a flat magnetic press element.

c. Lines of magnetic force tend to be concentrated in the corner portions and convex or concave portions having a large curvature along said periphery of the flat magnetic press element.

In the conventional magnetic medical treatment device as shown in said Figs. 10 and 11 the degree of concentration of lines of magnetic force onto each projection is low because each projection is arranged on a surface of said flat magnetic element near the center thereof and in addition lines of magnetic force are dispersed between said projections and a peripheral portion of said flat magnetic element, as shown in Fig. 11.

In addition, the conventional flat magnetic elements are made of magnetic material obtained by compression molding of powdery materials such as ferrite and then sintering the resulting molded products. However, such magnetic materials are rough to the touch and have an unattractive blackish external appearance which remarkably reduces their merchandise value. Moreover, the poor appearance of the magnetic elements means that a poor psychological effect is achieved which is a great disadvantage in medical treatments.

The principal object underlying the present invention is to modify a magnetic medical treatment device of the initially named kind so that the medical treatment effect is markedly improved, particularly by greater concentration of the lines of magnetic force. Furthermore, the medical treatment effect should be enhanced by modifying the surface of the magnetic element to exploit ionisation effects and/or to improve the appearance and thus the psychological effect of the magnetic element. The medical treatment effect should be improved still further by an improved construction of the sticking pad which makes the action of the magnetic element more effective.

In order to satisfy this object it is arranged in accordance with the present invention, that the bases of the projections touch a peripheral edge or edges of the element. In this way, lines of magnetic force from said flat magnetic press element are concentrated onto each said projection so that they may penetrate deep into the skin thus providing a sufficiently effective medical treatment by a combination of pressure-stimulation by said projections and concentration of the lines of magnetic force.

In one form of the magnetic medical treatment device of the present invention the magnetic element has the shape of a polygon and a projection is provided at each vertex of the polygon. This arrangement results in further concentration of the lines of magnetic force onto the positions of said projections thus further improving the magnetic medical treatment effect.

In another form of the magnetic medical treatment device of the present invention the magnetic element has radially projecting portions and a projection is formed at the radially outer extremity of each radially projecting portion.

In this arrangement depressions are formed between adjacent radially projecting portions around the periphery of the element, with the depressions extending from the front surface of the element adjacent the projections either partway through the element towards the rear surface, or all the way through the element. With these arrangements the lines of magnetic force are again better concentrated onto the positions of said projections, thus further improving the magnetic medical treatment effect.

A metal layer is preferably formed on the surface of said element, including said projections and preferably consists of gold, platinum or copper and the like. These metals, which are advantageously plated onto the surface of the magnetic element, improve its beauty and thus its merchandise value. Simultaneously, a psychological medical treatment effect is achieved through the grave metallic luster of said flat magnetic press element. The formation of metal ions of the metal layer can also prove beneficial to the medical treatment as will be later explained in more detail.

A plurality of the magnetic elements can also be provided on a sticking pad which then preferably has the form of a tape.

The sticking pad usefully comprises flexible sheet material. The flexible sheet material can usefully comprise interwoven warp and weft strands, with the warp strands comprising an elastic yarn covered by fibres.

In this way the sticking pad follows muscular movements and stretching of the same produces increased pressure of the projections on the patient's skin and thus improved stimulation. The flexible sheet material is advantageously provided with air spaces.

The invention will now be explained in more detail by way of example only and with reference to the accompanying drawings in which:

Fig. 1 is a plan view of a magnetic medical treatment device in accordance with the present invention,

Fig. 2 is a cross-sectional view through Fig. 1 taken along the lines II—II thereof showing the state of distribution of lines of magnetic force,

Fig. 3 is a perspective view showing another preferred embodiment of a magnetic medical treatment device in accordance with the present invention,

Fig. 4 and 5 are perspective views showing preferred embodiments of flat magnetic elements in accordance with the present invention,

Fig. 6 is a front view showing the state of distribution of lines of magnetic force around the flat magnetic element shown in Fig. 5,

Fig. 7 is an enlarged view showing the construction of sheet material preferably used for the sticking pads used to secure magnetic medical treatment devices in accordance with the present invention,

Figs. 8 and 9 show the magnetic medical treatment device of Fig. 1 in position on a patient,

Fig. 10 is a perspective view showing the conventional magnetic medical treatment device, and

Fig. 11 is a front view showing the state of distribution of lines of magnetic force around the conventional flat magnetic element of Fig. 10.

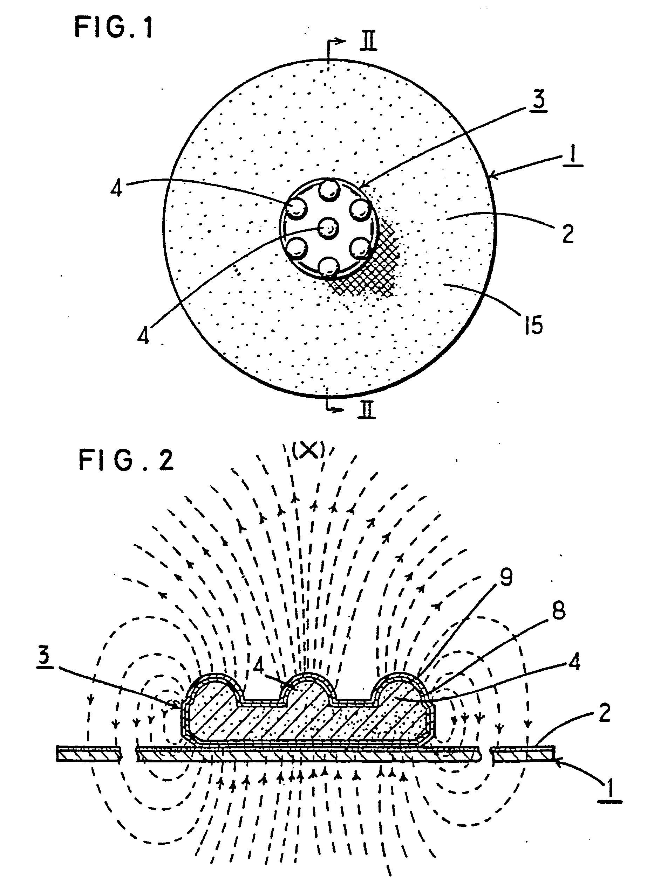

Referring firstly to Figs. 1 and 2 there can be seen a preferred embodiment in which a flat circular magnetic element 3 is mounted on the sticky surface 2 of a circular sticking pad 1 of small area such as adhesive tape and the like. Suitable magnetic elements are obtained by compression molding powdery materials such as ferrite and then sintering the resulting molded products followed by magnetizing. The flat magnetic press element 3 is provided with a plurality of small integral semicircular projections 4, at the central portion of its surface and along its periphery at angular intervals of 60°, for locally stimulating acupoints and meridians.

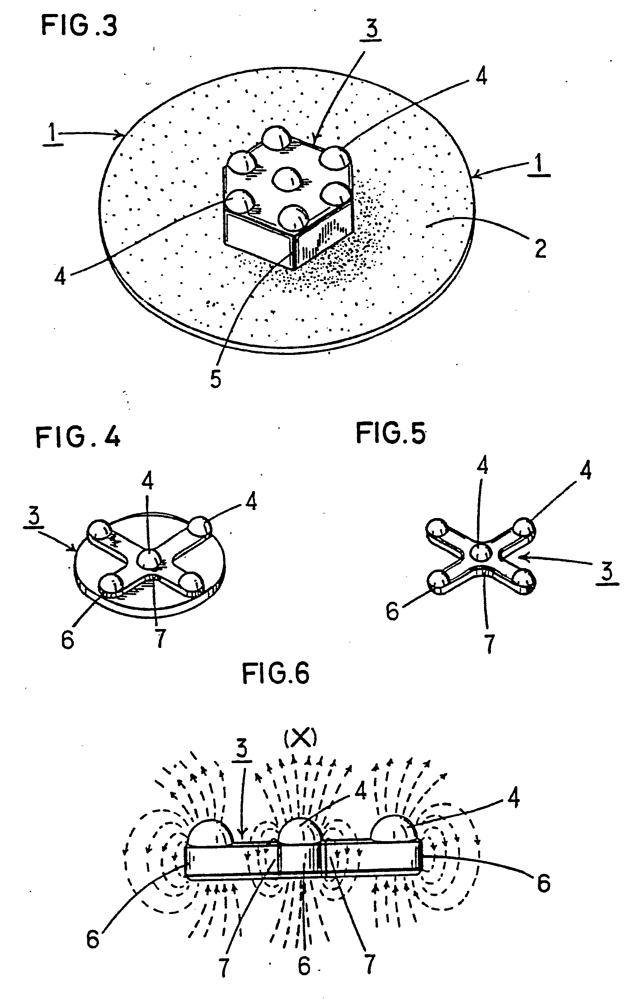

Fig. 3 shows a preferred embodiment in which the element 3 has the shape of a polygon and a projection 4 is provided at each vertex 5 of the polygon. Again the bases of the projections touch the peripheral edges of the element. The shape of said flat magnetic elements 3 may be hexagonal as shown but can also have other shapes such as pentagonal, octagonal and the like with a corresponding increase or decrease in the number of said projections.

Figs. 4 and 5 show preferred embodiments in which the magnetic elements 3 are provided with radially projecting portions, with a projection 4 being provided at the radially outer extremity of each radially projecting portion. The radially projecting portions are spaced at angular intervals of 90° and a further projection 4 is provided at the centre of each magnetic element. Depressions 7 are provided between the adjacent radially projecting portions around the periphery of the element to form convex portions 6 of large curvature at the positions corresponding to the positions of projections 4 mounted on said flat magnetic press element 3, i.e. at the radially outer extremities of the radially projecting portions and concave portions between said projections. In the Fig. 4 embodiment the depressions 7 extend from the front surface of the element 3 adjacent the projections 4 only part-way through the element towards the rear surface thereof.

In the Fig. 5 embodiment however the depressions 7 extend all the way through the element 3 from the front to the rear surface.

In the above described preferred embodiments the flat magnetic elements 3 are covered over the whole surface including the projections with a layer of metal 9 such as gold, platinum, silver, copper and the like through the intermediary of an intermediate layer 8 coated by means of cupric sulfate, as shown in Fig. 2.

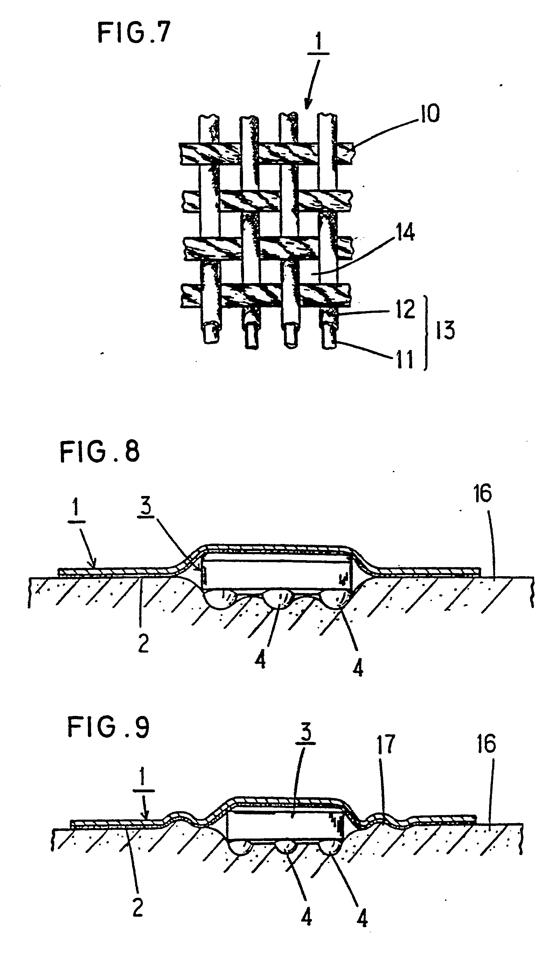

The sticking pad 1, as shown in Fig. 7, is made of a flexible sheet material provided with spaces 14 for passing air between flexible wefts 10 made of cotton, staple fiber and the like and flexible warps 13 which are prepared by covering elastic yarns 11, such as rubber yarns and the like, with fibers 12 such as cotton, staple fiber and the like and weaved alternately to and fro in a right-angled relation to said flexible wefts 10.

In addition, the adhesive layers 2 of the sticking pads 1 may include powdery silver or copper showing a sterilizing effect.

When a magnetic medical treatment device in accordance with the present invention is stuck on the skin 16 of a patient (see Fig. 8), the surface of the skin 16 is subjected to a pressing force from said magnetic element 3. The local stimulation by each said projection 4 in particular can provide an effective finger-pressure effect. Simultaneously, lines of magnetic force on said skin 16 from said magnetic element 3. However, the lines of magnetic force tend to concentrate on the projections of said magnetic element 3 rather than on its flat portion, along its periphery rather than on its flat portion and along its periphery rather than on the central portion of its surface. Referring now to Fig. 1 and Figs. 3 to 5, the projections 4 are arranged along a periphery of a surface of said flat magnetic press element 3 and thus lines of magnetic force X are concentrated on the positions of said projections 4, owing to the combined effect of the above described phenomena (see Fig. 2), to act on the affected part. Consequently a sufficiently effective medical treatment can be provided by the pressure stimulation effect of said projections 4 and by the action of the lines of magnetic force. Moreover, lines of magnetic force tend to concentrate on the angle portions (vertices) and the concave and convex portions of large curvature along a periphery of the magnetic elements 3. I.e., in the preferred embodiments shown in Figs. 3 to 5, lines of magnetic force tend to concentrate still more on the positions of said vertices 5 and the convex portions 6 at which said projections 4 are located around the periphery of said flat magnetic press element 3. In the preferred embodiments shown in Figs. 4 and 5, lines of magnetic force X are also concentrated on said concave portions of the depressions 7 adjacent to the central projection 4 and thus lines of magnetic force X act concentratively on the affected part (see Fig. 6).

The smooth surface of a metal layer obtained by plating 9 provides an agreeable touch to skin 16, and the grave and beautiful metallic luster of the metal layer 9 increases the psychological medical treatment effect. Thus, the combination of a finger-pressure effect and a magnetic medical treatment effect with a psychological medical treatment leads to an increased medical treatment effect. If said metal layer 9 comprises gold, in particular, ionization of the gold is accelerated by the substances such as ammonia, uric acid and the like which ooze out from the body onto the surface of skin 16 and the resulting gold ions exhibit their effect during the medical treatment of articular rheumatism. Moreover, when said metal layer 9 comprises silver or copper, ionization of the silver or copper is accelerated by the above described substances which ooze out from the body onto the surface of skin 16 and the resulting silver ions or copper ions may prevent the generation of rush owing to their sterilizing effect.

Furthermore, when said sticking pad 1 is made of flexible sheet materials, as shown in the preferred embodiments of the present invention, said sticking pad 1 is difficult to tear off from skin 16 because said sticking pad 1 expands and contracts in accordance with expansion and contraction of muscles and the generation of wrinkles. Moreover, when said sticking pad 1 is expanded, the force trying to contract the sticking pad 1 acts to press the magnetic element 3 against skin 16 thus reinforcing the stimulation due to pressure.

## Claims

1. A magnetic medical treatment device comprising a flat magnetic element (3) mounted upon the sticky surface of a sticking pad (1), the flat element (3) being provided with a plurality of projections (4) facing away from the sticky surface, characterised in that the bases of the projections touch a peripheral edge (or edges) of the element (3).

2. A magnetic medical treatment device in accordance with claim 1 and characterised in that the element (3) has the shape of a polygon and in that a projection (4) is provided at each vertex (5) of the polygon.

3. A magnetic medical treatment device in accordance with claim 1 and characterised in that said element (3) has radially projecting portions and in that a projection (4) is formed at the radially outer extremity of each radially projecting portion.

4. A magnetic medical treatment device in accordance with claim 3 and characterised in that depressions (7) are formed between adjacent radially projecting portions (Figs. 4 and 5) around the periphery of the element, with the depressions (7) extending partly from the front surface of the element adjacent the projections either part-way through the element (Fig. 4) towards the rear surface or all the way through the element (Fig. 5).

5. A magnetic medical treatment device in accordance with any one of the preceding claims wherein a further projection (4) is provided at the centre of said element.

6. A magnetic medical treatment device in accordance with any one of the preceding claims and characterised in that a metal layer (9) is formed the surface of said element (3), including said projections (4) and preferably consists of gold, silver, platinum or copper.

7. A magnetic medical treatment device in accordance with claim 6 and characterised in that said metal layer (9) is applied to said surface via an intermediate layer (8).

8. A magnetic medical treatment device in accordance with any one of the preceding claims and characterised in that a plurality of said elements (3) are provided on a sticking pad preferably in the form of a tape.

9. A magnetic medical treatment device in accordance with any one of the preceding claims and characterised in that said sticking pad comprises flexible sheet material.

10. A magnetic medical treatment device in accordance with claim 9 and characterised in that said flexible sheet material comprises interwoven warp and weft threads (13, 10) with the warp threads comprising an elastic yarn (11) covered by fibres (12).

11. A magnetic medical treatment device in accordance with either of claims 6 and 7 and wherein said flexible sheet material includes air spaces (14).

**Patentansprüche**

1. Magnetische Einrichtung zur medizinischen Behandlung mit einem an der Klebfläche eines Klebekissens (1) angebrachten ebenen magnetischen Element (3), das mit einer Vielzahl von der Klebfläche weggerichteten Erhebungen (4) versehen ist, dadurch gekennzeichnet, daß die Grundabschnitte der Erhebungen eine Umfangskante (oder Umfangskanten) des Elementes (3) berühren.

2. Magnetische Einrichtung zur medizinischen Behandlung nach Anspruch 1, dadurch gekennzeichnet, daß das Element (3) die Gestalt eines Vielecks besitzt und daß in jedem Eck (5) des Vielecks eine Erhebung vorgesehen ist.

3. Magnetische Einrichtung zur medizinischen Behandlung nach Anspruch 1, dadurch gekennzeichnet, daß Element (3) radial abstehende Abschnitte besitzt und daß eine Erhebung (4) im radial äußeren Endbereich jedes radial abstehenden Abschnittes ausgebildet ist.

4. Magnetische Einrichtung zur medizinischen Behandlung nach Anspruch 3, dadurch gekennzeichnet, daß zwischen benachbarten radial abstehenden Abschnitten Vertiefungen (7) ausgebildet sind (Fig. 4 und 5) un den Umfang des Elementes, wobei sich die Vertiefungen (7) teilweise von der Vorderfläche des Elementes in Nachbarschaft zu den Erhebungen entweder teilweise durch das Element erstrecken (Fig. 4) gegen die Rückfläche hin, oder durch das gesamte Element hindurch (Fig. 5).

5. Magnetische Einrichtung zur medizinischen Behandlung nach einem der vorangehenden Ansprüche, bei der eine weitere Erhebung (4) im Zentrum des Elementes vorgesehen ist.

6. Magnetische Einrichtung zur medizinischen Behandlung nach einem der vorangehenden Ansprüche, dadurch gekennzeichnet, daß an der Oberfläche des Elementes (3) einschließlich der Erhebungen (4) eine bevorzugt aus Gold, Silber, Platin oder Kupfer bestehende Metallschicht (9) ausgebildet ist.

7. Magnetische Einrichtung zur medizinischen Behandlung nach Anspruch 6, dadurch gekennzeichnet, daß die Metallschicht (9) über eine Zwischenschicht (8) an der Oberfläche aufgebracht ist.

8. Magnetische Einrichtung zur medizinischen Behandlung nach einem der vorangehenden Ansprüche, dadurch gekennzeichnet, daß eine Vielzahl der Elemente (3) an einem bevorzugt bandförmig ausgebildeten Klebekissen vorgesehen sind.

9. Magnetische Einrichtung zur medizinischen Behandlung nach einem der vorangehenden Ansprüche, dadurch gekennzeichnet, daß das Klebekissen flexibles Schichtmaterial umfaßt.

10. Magnetische Einrichtung zur medizinischen Behandlung nach Anspruch 9, dadurch gekennzeichnet, daß das flexible Schichtmaterial miteinander verwebte Kett- und Schußfäden (13, 10) enthält, wobei die Kettfäden ein durch Fasern (12) bedecktes elastisches Garn (11) umfassen.

11. Magnetische Einrichtung zur medizinischen Behandlung nach einem der Ansprüche 6 oder 7, bei der das flexible Schichtmaterial Luft-Zwischenräume (14) enthält.

**Revendications**

1. Dispositif magnétique de traitement médical comportant un élément magnétique plat (3) monté sur la surface adhésive d'un tampon collant (1), l'élément plat (3) étant muni d'une pluralité de bossages (4) regardant à l'opposé de la surface adhésive, caractérisé en ce que les bases des bossages touchent un (ou des) bord(s) périphérique(s) de l'élément (3).

2. Dispositif magnétique de traitement médical selon la revendication 1 et caractérisé en ce que l'élément (3) a la forme d'un polygone et en ce qu'un bossage (4) est prévu à chaque sommet (5) du polygone.

3. Dispositif magnétique de traitement médical selon la revendication 1 et caractérisé en ce que ledit élément (3) a des parties dépassant radialement et en ce qu'un bossage (4) est formé à l'extrémité radialement extérieure de chaque partie dépassant radialement.

4. Dispositif magnétique de traitement médical selon la revendication 3 et caractérisé en ce que des dépressions (7) sont formées entre des parties dépassant radialement adjacentes (figures 4 et 5) sur le pourtour de l'élément, les dépressions (7) s'étendant à partir de la surface frontale de l'élément au voisinage des bossages vers la surface arrière soit sur une partie de l'épaisseur de l'élément (figure 4) soit de part en part de l'élément (figure 5).

5. Dispositif magnétique de traitement médical selon l'une quelconque des revendications précédentes caractérisé en ce qu'un bossage supplémentaire (4) est prévu au centre dudit élément.

6. Dispositif magnétique de traitement médical selon l'une quelconque des revendications précédentes et caractérisé en ce qu'une couche métallique (9) est formée sur la surface dudit élément (3), y compris lesdits bossages (4) et est de préférence en or, argent, platine ou cuivre.

7. Dispositif magnétique de traitement médical selon la revendication 6 caractérisé en ce que ladite couche métallique (9) est appliquée sur ladite surface au moyen d'une couche intermédiaire (8).

8. Dispositif magnétique de traitement médical selon l'une quelconque des revendications précédentes et caractérisé en ce qu'une pluralité desdits éléments (3) sont prévus sur un tampon collant de préférence en forme de ruban.

9. Dispositif magnétique de traitement médical selon l'une quelconque des revendications précédentes et caractérisé en ce que ledit tampon collant comporte une matière en feuille souple.

10. Dispositif magnétique de traitement médical selon la revendication 9 et caractérisé en ce que ladite matière en feuille souple comporte des fils de chaîne et de trame entrecroisés (13, 10), les fils de chaîne comportant un fil élastique (11) recouvert de fibres (12).

11. Dispositif magnétique de traitement médical selon l'une des revendications 6 et 7 et caractérisé en ce que ladite matière en feuille souple comporte des aérations (14).

FIG.1

FIG.2

# FIG.3

# FIG.4

# FIG.5

# FIG.6

(X)

FIG.7

FIG.8

FIG.9

## FIG.10

## FIG .11